# EUROPEAN PATENT APPLICATION

(11) **EP 0 635 573 A2**
(43) Date of publication of application: **25.01.1995**
(21) Application number: 94304707.6
(22) Date of filing: 28.06.1994
(51) Int. Cl.: C12N 15/62, C12P 21/02, C07K 14/605

(54) **Process for the preparation of native human glicentin**

(30) Priority: 30.06.1993 JP 160977/93; 28.12.1993 JP 334126/93
(71) Applicant: NISSHIN FLOUR MILLING CO., LTD., Chuo-ku, Tokyo 103 (JP)
(72) Inventor: Sato, Takeya, c/o Nisshin Flour Milling Co., Ltd., Chuo-ku, Tokyo (JP); Natori, Yohei, c/o Nisshin Flour Milling Co. Ltd., Ohimachi, Iruma-gun, Saitama-ken (JP)
(74) Representative: Marshall, Monica Anne

(57) **Abstract**

Disclosed is a process for the preparation of native human glicentin, which comprises producing by recombinant gene technology a fused polypeptide composed of human glicentin having an extra peptide moiety linked to the amino terminus and cleaving the extra peptide moiety in the fused polypeptide, wherein the peptide moiety upon cleavage is designed to constitute even-numbered amino acids and the cleavage of the extra peptide moiety is carried out by hydrolysis with cathepsin C. T7S10 peptide is used as the peptide moiety.

## Description

### FIELD OF THE INVENTION

This invention relates to a process for the preparation of native human glicentin useful for pharmaceutical applications such as antidiabetics.

### BACKGROUND OF THE INVENTION

Glicentin is a gastrointestinal peptide hormone composed of 69 amino acids, plays an important role in the physiology of gastrointestines and is used as pharmaceuticals, e.g., antidiabetics as disclosed in Japanese Patent Kokai 4-185066 (corresponding to EPA 0586812). Glicentin was isolated and purified from porcine small intestine for the first time by F. Sundby et al [F. Sundby, et al., Horm. Metab. Res. Vol. 18, 366-371 (1976)] and its structure was then identified by A. Thim and A. J. Moody [A. Thim and A. J. Moody, Regul. Pept. Vol. 2, 139-150 (1981)]. Afterward, the structures of glicentin genes from human and several animal species were elucidated. As a result, it was revealed that the amino acid sequence of human glicentin is distinct from that of other animal species. Because of difficult availability of human organs, human glicentin has never been isolated or purified.

It is considered that methods for the production of glicentin include a method for the extraction from animal organs and a method by chemical synthesis. The above extraction method requires a great deal of such organs, because of the glicentin content in animal oragns being quite low. It is possible to isolate and purify glicentin from such organs as porcine ones that are abundantly available. However, the resultant purified glicentin is peculiar to the animal species and different from human glicentin. Extraction from human organs is not feasible because of their availability being almost impossible. The method by chemical synthesis cannot provide glicentin in large quantities at low price, since the reaction yield is low due to its long amino acid sequence and expensive reagents are used.

We have previously developed a technique of preparing human glicentin which comprises cultivating a microorganism transformed with a vector having a synthetic DNA inserted thereinto which codes for the human glicentin amino acid sequence (see, "Proceedings of the Thirteenth Gut Hormone Conference", edited by JAPANESE SOCIETY OF GUT HORMONES and published by IGAKU TOSHO SHUPPAN LTD. in 1992, Vol. 11, pp. 394-401). However, the resultant glicentin contains additional methionine at the amino terminus, which is different from native glicentin.

In the production of the protein by recombinant gene technology, there is prepared a peptide containing additional methionine at the amino terminus encoded by the translation initiation codon ATG of the gene. The use of the protein thus produced for drugs, foods or the like requires to remove the additional methionine from the protein, since methionine may bring about an antigen.

The methods of producing native peptide by removal of methionine at the amino terminus include a chemical process by the site-specific cleavage at the methionine of the fused peptide with cyanogen bromide and an enzymatic process involving the preparation of a fused peptide having a recognition sequence for a particular site-specific proteinase inserted between the appropriate extra and desired peptide segments, followed by the site-specific hydrolysis with the enzyme. However, the chemical process will hydrolyze the glicentin at the methionine site due to the presence of methionine in the glicentin amino acid sequence. In the case of the enzymatic process, lots of by-products are produced with reduced yield of the desired glicentin, due to the presence in the glicentin amino acid sequence of sites more susceptible to hydrolysis with the proteinase used. Furthermore, it is also conceivable to produce a peptide of interest from its fused peptide with another peptide by removal of the peptide attached to the desired peptide using aminopeptidases or carboxypeptidases which so hydrolyze the fused peptide as to remove amino acids, one by one, from the amino terminus or carboxyl terminus, respectively. These enzymes, however, can not be used in processes for industrial application, since due to their low substrate-specificity a delicate adjustment of reaction conditions which is extremely difficult is required if the reaction is to be terminated at the desired site. Thus, known processes did not allow any satisfactory production of native glicentin.

### SUMMARY OF THE INVENTION

Under such circumstances, we have made continuing investigations in an effort to prepare effectively native human glicentin and found that this aim can be attained by a process which comprises producing by recombinant gene technology a fused polypeptide wherein an extra peptide moiety is linked to the amino terminus of glicentin and hydrolyzing the fused polypeptide with cathepsin C to remove or cleave the extra peptide moiety therefrom, the extra peptide moiety being designed to constitute even-numbered amino acids upon cleavage. The present invention is based on the findings that glicentin has arginine at the amino terminus, cathepsin C hydrolyzes a protein to remove or cleave amino acids in pairs from the amino terminus of the protein, while the reaction ceases and no further hydrolysis takes place wherever a basic amino acid appears at the amino terminus of the protein.

According to the present invention, there is provided a process for the preparation of native human glicentin, which comprises producing by recombinant gene technology a fused polypeptide composed of human glicentin having an extra peptide moiety linked to the amino terminus and cleaving the extra peptide moiety in the fused polypeptide, wherein the peptide moiety upon cleavage is designed to constitute even-numbered amino acids and the cleavage of the extra peptide moiety is carried out by hydrolysis with cathepsin C.

### BRIEF DESCRIPTION OF DRAWINGS

Fig. 1 is a flow diagram illustrating the process steps for the construction of plasmid pGL125.

Fig. 2 is a flow diagram illustrating the process steps for the construction of plasmid pGL144.

Fig. 3 is a flow diagram illustrating the process steps for the construction of plasmid pGL146.

Fig. 4 is a flow diagram illustrating the process steps for the construction of plasmid pGL145.

Fig. 5 is a HPLC chromatogram of hydrolyzate with lysyl endopeptidase of purified product from cathepsin C treatment of a fused glicentin of the present invention.

### DETAILED DESCRIPTION OF THE INVENTION

In the embodiment of the present invention, an expression vector is constructed by linking a gene for a fused polypeptide composed of glicentin having an extra peptide moiety attached to the amino terminus, to the downstream from a promoter capable of being expressed in a host cell. The vector is used to transform a cell capable of expressing the gene. The transformed cell is cultured and if necessary, subjected to induced expression of the gene, thereby producing the fused polypeptide. Subsequently, the fused polypeptide is hydrolyzed with cathepsin C to remove or cleave the extra peptide moiety, thus providing a desired native human glicentin. The native human glicentin has the following amino acid sequence:

Arg-Ser-Leu-Gln-Asp-Thr-Glu-Glu-Lys-Ser-Arg-Ser-Phe-Ser-Ala-
Ser-Gln-Ala-Asp-Pro-Leu-Ser-Asp-Pro-Asp-Gln-Met-Asn-Glu-Asp-
Lys-Arg-His-Ser-Gln-Gly-Thr-Phe-Thr-Ser-Asp-Tyr-Ser-Lys-Tyr-
Leu-Asp-Ser-Arg-Arg-Ala-Gln-Asp-Phe-Val-Gln-Trp-Leu-Met-Asn-
Thr-Lys-Arg-Asn-Arg-Asn-Asn-Ile-Ala

The promoters used for the gene expression can include any sequences capable of being recognized in host cells, φ10 promotor from bacteriophage T7 being preferred. The host cells which may be used in the invention include any cells capable of recognizing the promoter. Escherichia coli is preferred.

Peptides which can be used for fusing with glicentin include any peptides which are designed to constitute even-numbered amino acids upon cleavage of the fused polypeptide. T7S10 peptide is preferably used, because of providing increased yield of the fused polypeptide. A further amino acid sequence may be inserted between glicentin and the peptide to be fused therewith. Preferably, the peptides to be fused with glicentin consist of not greater than 20 amino acids from the standpoint of hydrolysis efficiency by cathepsin C. The hosts used in the invention include any Escherichia coli. If the glicentin gene is to be expressed using φ10 promoter, T7 RNA polymerase should be produced in Escherichia coli. The requirements for allowing T7 RNA polymerase to be produced in Escherichia coli can be met, for example, by infection of Escherichia coli with T7 bacteriophage, by use of Escherichia coli possessing a plasmid carrying a T7 RNA polymerase gene or by use of Escherichia coli having a T7 RNA polymerase gene integrated in a chromosome. For the purpose of controlling the expression of T7 RNA polymerase gene it is preferable to use Escherichia coli having integrated in the chromosome a T7 RNA polymerase gene so constructed as to be controllably expressible in Escherichia coli. As Escherichia coli strains can preferably be used Escherichia coli HMS174(DE3), Escherichia coli HMS174(DE3)pLysS, Escherichia coli HMS174(DE3)pLysE, Escherichia coli BL21(DE3), Escherichia coli BL21(DE3)pLysS, and Escherichia coli BL21(DE3)pLysE. These strains are commercially available to those skilled in the art.

In the production of the fused glicentin, the transformed cell is subjected to aerobic culture in a culture medium containing carbon and nitrogen sources, trace nutrients, trace metal ions, etc., and if necessary, subjected to induced expression of T7 RNA polymerase. The culture media include any media in which Escherichia coli is able to produce glicentin, M9ZB being preferable. If necessary, other substances may be added.

The fused glicentin accumulated in the cell may be purified by combinations of conventional processes used for purifying peptides. One preferred process is mentioned in "Proceedings of The Thirteenth Gut Hormone Conference" as cited above.

In the cleavage of the extra peptide moiety by hydrolysis with cathepsin C to produce native human glicentin, the peptide moiety is required to constitute even-numbered amino acids. If any basic amino acid is optionally present in the peptide moiety, it should be located at a position in even numbers from the amino terminus. Further, it is required that no proline is contained in the amino acids at a position in odd numbers from the amino terminus.

In the process of the present invention, it is required that the amino acid number of the peptide moiety upon cleavage should be even. To achieve this effect, a variety of methods are employed. One of the methods includes the construction of a gene encoding the following amino acid sequence Met-A-glicentin
wherein Met stands for methionine encoded by the translation initiation codon ATG and A stands for a peptide consisting of an even number of amino acids, the amino terminus of which is selected from alanine, glycine, serine, valine or threonine to produce the fused polypeptide, by which the amino terminal methionine is removed through processing by a producing strain, thus rendering even the amino acid number of the peptide moiety attached to the glicentin. Another method includes the construction of a gene encoding the following amino acid sequence Met-B-glicentin
wherein Met is as defined above and B stands for either an amino acid selected from arginine, asparagine, aspartic acid, glutamine, glutamic acid, isoleucine, leucine, lysine, tryptophan, tyrosine, phenylalanine or hystidine, or a peptide consisting of an odd number of amino acid, the amino terminus of which is selected from arginine, asparagine, aspartic acid, glutamine, glutamic acid, isoleucine, leucine, lysine, tryptophan, tyrosine, phenylalanine or histidine to produce the fused polypeptide, by which the amino terminal methionine is not removed through processing by a producing strain, thus rendering even the amino acid number of the peptide moiety attached to the glicentin.

The hydrolysis of fused glicentin with cathepsin C may be carried out under those conditions conventionally employed in reactions with the enzyme. If necessary, such protease inhibitors as leupeptin, antipine, chymostatin and E-64 may be added to the reaction solution in order to inhibit the activity of any proteinase.

The invention is further illustrated by the following examples.

### EXAMPLE 1

Construction of expression vector

An expression vector was constructed in which a fused-glicentin gene was linked downstream of T7φ10 promoter. A series of experimental operations was carried out in accordance with conventional recombinant gene experimental methods.

In order to carry out the linkage of the glicentin gene DNA, an Stu I recognition sequence was inserted into vector plasmid pET-3a (available from Novagen) downstream of the Bam HI recognition sequence.

Two oligonucleotides of the following sequences: 
5'-GATCCTTAGCGTAGGCCTT-3' (SEQ ID NO: 1 in the Sequence
Listing), and
5'-GATCAAGGCCTACGCTAAG-3' (SEQ ID NO: 2 in the Sequence
Listing)
were synthesized, using a Cyclone Plus DNA synthesizer manufactured by Milligen/Biosearch and synthetic reagents also supplied by the same manufacturer, in accordance with the accompanying operation manual. These oligonucleotides were purified by use of an Oligo-Pak™ manufactured by Milligen/Biosearch and the accompanying oligonucleotide purification manual. These procedures were used to perform the synthesis and purification of all the oligonucleotides to be used in the experiments which follow. Five µg of each oligonucleotide was phosphorylated with T4 polynucleotide kinase manufactured by Takara Shuzo under the reaction conditions as commended by the manufacturer. The two reaction solutions were combined together, kept at 90°C for 5 minutes and then cooled down to 30°C over a period of 3 hours to carry out the annealing of oligonucleotides. Plasmid pEt-3a (see, F. W. Studier et al., Methods in Enzymology, Vol. 185, pp. 60-89 (1990)) was hydrolyzed with Bam HI and then dephosphorylated with E. coli alkaline phosphatase manufactured by Takara Shuzo under the reaction conditions as commended by the manufacturer. The resultant linear plasmid and the annealed oligonucleotides were ligated together using a DNA ligation kit manufactured by Takara Shuzo in accordance with the protocol as commended by the manufacturer. The ligated DNA was used to transform E. coli JM109 using the calcium chloride technique and those transformants were selected which grew on LB agar culture media containing ampicillin at a concentration of 50 µg/ml. The resultant transformant was cultivated overnight in an LB medium containing ampicillin at a concentration of 50 µg/ml, followed by extraction and purification of plasmids from the cultured cells. The resultant plasmids were subjected to hydrolysis with Stu I followed by agarose gel electrophoresis to select those plasmids which possess a cleavage site for this enzyme. The nucleotide sequence of these plasmids was determined by the dideoxy method using a Sequenase version 2.0 kit manufactured by United States Biochemical Corporation in accordance with the protocol as commended by the manufacturer. The newly obtained plasmid was designated pET110. The pET110 was confirmed to have newly an Stu I recognition sequence downstream of the Bam HI recognition sequence. The nucleotide sequence between the Bam HI and Stu I recognition sequences is shown as SEQ ID NO: 3 in the Sequence Listing.

The Pst I recognition sequence present downstream of the glicentin gene in pGL5 (see "Proceedings of the Thirteenth Gut Hormone Conference", edited by JAPANESE SOCIETY OF GUT HORMONES and published by IGAKU TOSHO SHUPPAN LTD. in 1992, Vol. 11, pp. 394-401) was converted into an Sma I recognition sequence.

Two oligonucleotides of the following sequences: 
5'-CATGGCCCGGGACAGCACA-3' (SEQ ID NO: 4 in the Sequence
Listing) and
5'-AGCTTGTGCTGTCCCGGGC-3' (SEQ ID NO: 5 in the Sequence
Listing)
were synthesized and then subjected to purification, phosphorylation and annealing in the same manner as described above. pGL5 was hydrolyzed with Nco I and Hind III and the reaction products were subjected to agarose gel electrophoresis. The desired fragments were purified by extraction from the gel of the ca. 4 kb band. The thus purified linear plasmid was ligated with the annealed oligonucleotides. The ligated DNA was used to transform E. coli JM109 in the same manner as in the construction of the pET110. Again in the same manner as with the pET110 plasmids were extracted and purified from the transformant. The nucleotide sequence of the resultant plasmids were hydrolyzed with Sma I to select those plasmids in which a restriction site for this enzyme was present. The nucleotide sequence of these plasmids was determined to select a plasmid having the desired sequence, which was designated pGL44. In the newly obtained plasmid pGL44, the Pst I recognition sequence in 3'-non coding region of glicentin gene had been deleted and an Sma I recognition site added newly. The nucleotide sequence of the Nco I - Hind III region of the pGL44 including the new Sma I site placed downstream of the glicentin gene is shown as SEQ ID NO: 6 in the Sequence Listing.

The 5'-end region of the glicentin gene of pGL44 was modified.

Two oligonucleotides of the following sequences: 
5'-AATTCAGATCTATCGAAGGTCGACGTTCTCTGCA-3'
(SEQ ID NO: 7 in the Sequence Listing) and
5'-GAGAACGTCGACCTTCGATAGACTG-3' (SEQ ID NO: 8 in the
Sequence Listing)
were synthesized and then subjected to purification, phosphorylation and annealing in the same manner as described above. pGL44 was hydrolyzed with Eco RI and Pst I, dephosphorylated in the same manner as described above, and then ligated with the annealed oligonucleotides. The annealed DNA was used to transform E. coli JM109. Plasmids were extracted from the transformant and treated with Bgl II to select those plasmids which came to be able to be cleaved with this enzyme. The nucleotide sequence of these plasmids was identified and a plasmid having the desired nucleotide sequence was designated pGL122. The pGL122 was confirmed to be that having the synthetic oligonucleotides inserted into the Eco RI - Pst I site. The nucleotide sequence of the modified site in the pGL122 is shown as SEQ ID NO: 9 in the Sequence Listing.

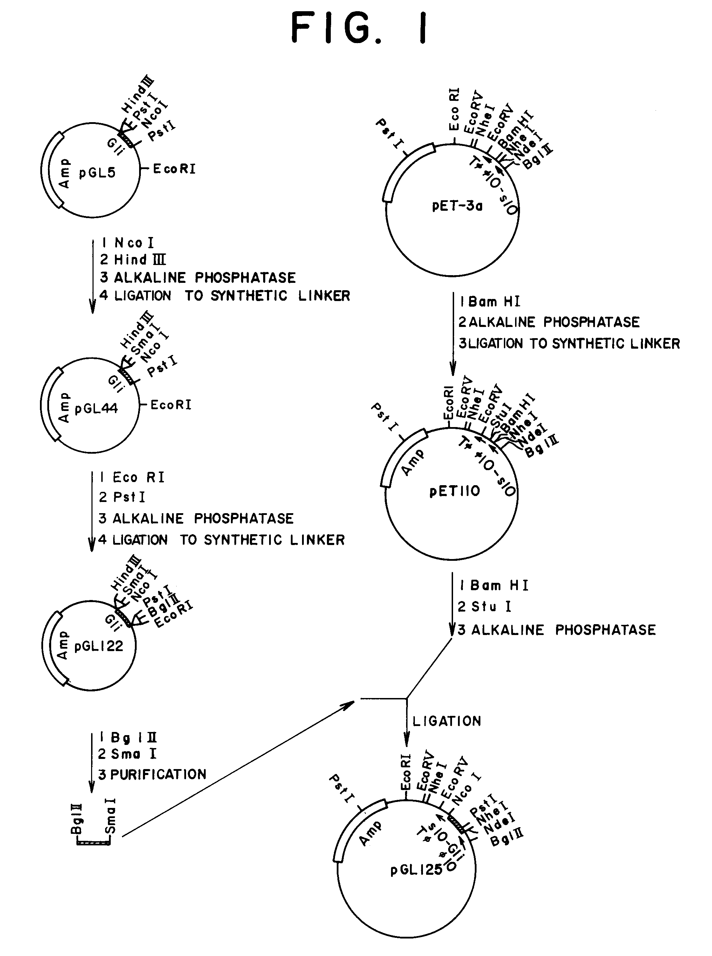

pGL122 was hydrolyzed with Bgl II and Sma I and the glicentin gene-containing DNA fragment was purified. pET110 was hydrolyzed with Bam HI and Stu I and then dephosphorylated. The product was mixed with the glicentin gene-containing DNA fragment and ligated thereto by using T4 DNA ligase. Transformation of E. coli JM109 was performed by mixing the reaction product therewith to give a new plasmid pGL125. The pGL125 is an expression vector in which downstream of the T7φ10 promoter is linked a gene encoding the T7s10 peptide-inserted amino acid sequence-glicentin fused peptide. The process steps for the construction of pGL125 are schematically shown in Fig. 1.

### Construction of gene encoding Met-Arg-fused glicentin

### A) Construction of pGL144

pUC18 was cleaved with restriction enzyme Dra I and then subjected to agarose gel electrophoresis to purify the ampicillin-resistance gene-containing fragment. pGL125 was cleaved with restriction enzyme Dra I and then subjected to agarose gel electrophoresis to purify the ampicillin-resistance gene-free fragment. These fragments were subjected to ligation using T4 DNA ligase. The reaction product was introduced into competent E. coli JM109 cells and the transformant was selected on an ampicillin-containing selection medium.

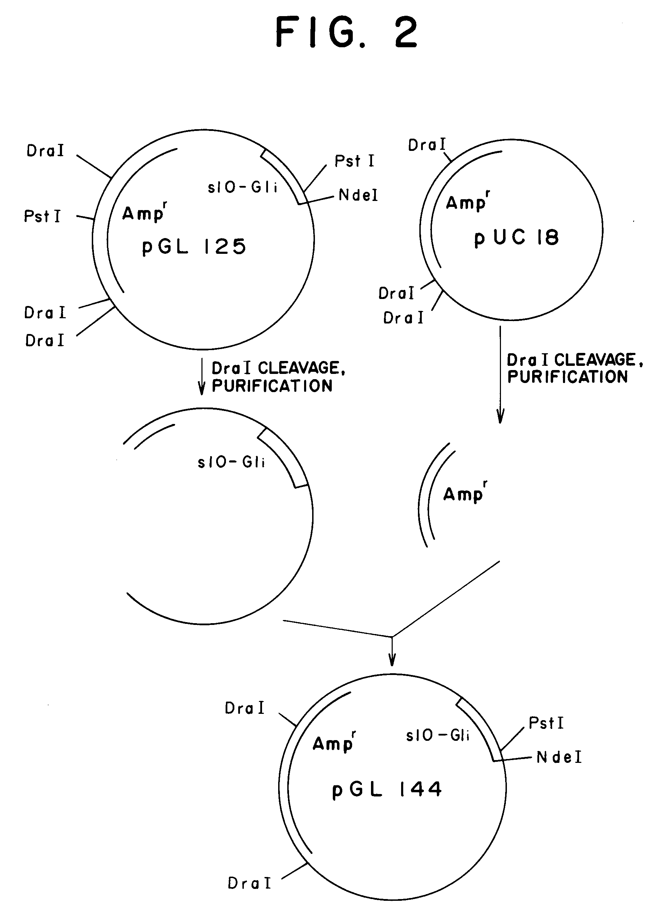

Plasmid was extracted from the resultant transformant in accordance with a conventional method and a restriction enzyme cleavage map was prepared to confirm the construction of the desired plasmid, which was designated pGL144. The process steps for the construction of pGL144 are schematically shown in Fig. 2.

### B) Construction of pGL146

The pGL144 constructed in A) above was cleaved with restriction enzymes Nde I and Pst I, and a synthetic linker was inserted thereinto. The sequences constituting the synthetic linker are shown in the following:

5'-TATGCGTCGTTCCCTGCA-3' (SEQ ID NO: 10 in the Sequence
Listing) and
5'-GGGAACGACGCA-3' (SEQ ID NO: 11 in the Sequence Listing)

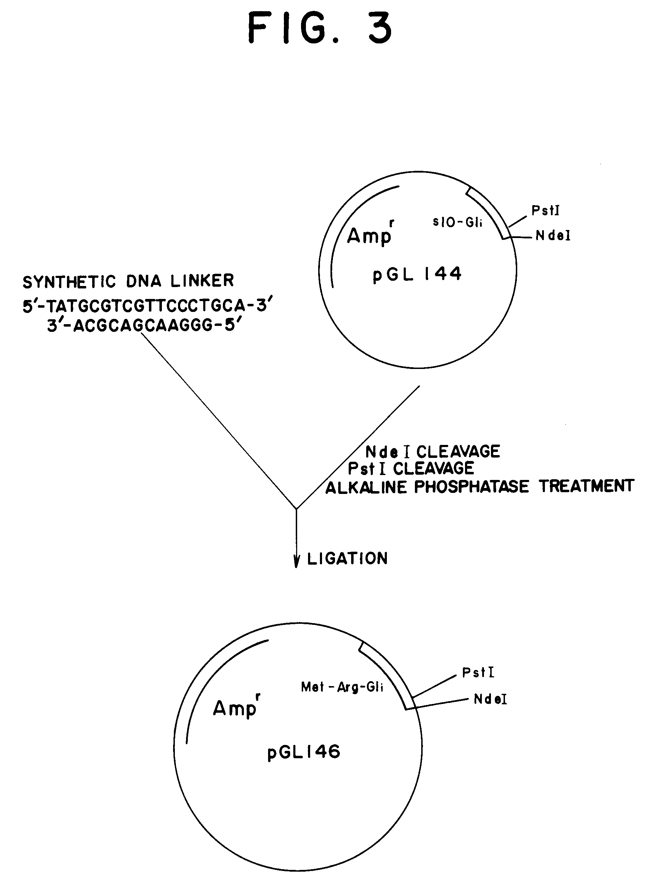

Two µg of each of the synthetic DNAs was separately phosphorylated with T4 polynucleotide kinase. The two reaction mixtures were combined and the resultant mixture was heated at 94°C for 5 minutes and then cooled down to room temperature over a period of 1 hour for annealing. Using a DNA ligation kit manufactured by Takara Shuzo, the thus prepared synthetic DNA linker was ligated to a linear pGL144 obtained by cleavage with restriction enzymes Nde I and Pst I followed by purification by means of agarose gel electrophoresis. The thus ligated DNA was introduced into competent E. coli JM109 cells and the transformant selection was carried out on an ampicillin-containing selection medium. The plasmid was extracted from the resultant transformant in the conventional manner to confirm, using the dideoxy method of Sanger et al., that it had the desired base sequence. This plasmid was designated pGL146. The process steps for the construction of pGL146 are schematically shown in Fig. 3.

### EXAMPLE 2

Construction of gene encoding s10-peptide-fused glicentin

A gene was constructed which encodes a fused peptide composed of glicentin and an eleven-amino acid peptide containing methionine corresponding to the N-terminal translation initiation codon ATG of the s10 peptide described in F. W. Studier, et al., Methods in Enzymology, Vol. 185, p. 74 (1990). The pGL144 constructed in Example 1-A) was cleaved with restriction enzymes Nde I and Pst I, and a synthetic linker was inserted thereinto. The sequences constituting the synthetic DNA linker are shown in the following:

5'-TATGGCTAGCATGACTGGTGGACAGCAAATGGGTCGTTCCCTGCA-3'
(SEQ ID NO: 12 in the Sequence Listing) and
5'-GGGAACGACCCATTTGCTGTCCACCAGTCATGCTAGCCA-3'
(SEQ ID NO: 13 in the Sequence Listing)

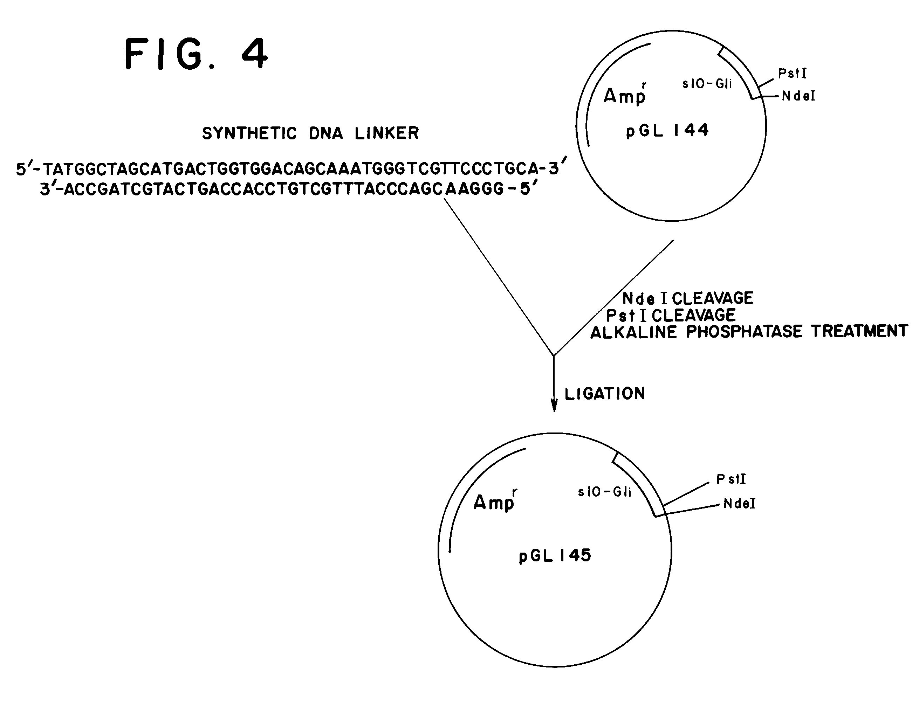

Two µg of each of the synthetic DNAs was separately phosphorylated with T4 polynucleotide kinase. The two reaction solutions were combined together and the resultant mixture was heated at 95°C for 5 minutes and then cooled down to room temperature over a period of 1 hour for annealing. Using a DNA ligation kit manufactured by Takara Shuzo, the thus prepared synthetic DNA linker was ligated to a linear pGL144 obtained by cleavage with restriction enzymes Nde I and Pst I followed by purification by means of agarose gel electrophoresis. The thus ligated DNA was introduced into competent E. coli JM109 cells and the transformant selection was carried out on an ampicillin-containing selection medium. The plasmid was extracted from the resultant transformant in the conventional manner to confirm, using the dideoxy method of Sanger et al., that it had the desired sequence. This plasmid was designated pGL145. The process steps for the construction of pGL145 are shown in Fig. 4.

### EXAMPLE 3

Production of fused glicentin

E. coli HMS174(DE3)pLysS was transformed with pGL146 to give a transformant with the capability of producing the fused glicentin. This transformant was used to inoculate a culture medium containing, per liter thereof, 10 g of NZ-amine, 5 g of NaCl, 1 g of NH₄Cl, 3 g of KH₂PO₄, 6 g of Na₂HPO₄, 4 g of glucose and 264 mg of MgSO₄ 7H₂O and then shake-cultured therein at 37°C. At a point of time when the cell density reached A₅₅₀=0.6 was added isopropyl-β-D-thiogalactopyranoside to a final concentration of 0.5 mM, followed by further 2.5 hours of cultivation. When measured by enzyme immunoassay using an antibody directed to the amino-terminus and that directed to the carboxyl-terminus of glicentin ("Proceedings of the Thirteenth Gut Hormone Conference", edited by JAPANESE SOCIETY OF GUT HORMONES and published by IGAKU TOSHO SHUPPAN LTD. in 1992, Vol. 11, pp. 358-363), the amount of the fused protein accumulated in the cells was 30 mg as glicentin per liter of culture. From the cells in one liter of the cultures was purified the fused glicentin by using the procedure as described in Shouka-kan Hormone (XI), edited by "Proceedings of the Thirteenth Gut Hormone Conference", edited by JAPANESE SOCIETY OF GUT HORMONES and published by IGAKU TOSHO SHUPPAN LTD. in 1992, Vol. 11, pp. 394-401) to give 7.3 mg of pure preparation.

By applying the pure preparation to a peptide sequencer, the first five amino-terminal amino acids were determined to have the sequence Met-Arg-Arg-Ser-Leu-. Thus the preparation was found to be a fused protein composed of native glicentin with two additional amino acids attached as designed at the amino-terminus.

### EXAMPLE 4

E. coli HMS174(DE3)pLysS was transformed with pGL145 to give a transformant with the capability of producing the fused glicentin. Under the same conditions as in Example 3, this transformant was cultured and the fused glicentin purified. Again in the same manner as described in Example 3, the amounts of the fused glicentin accumulated in the cells and the purified glicentin were measured. The amount of the fused glicentin accumulated in the cells was 90 mg per liter of culture and that of the purified glicentin was 22 mg.

By applying the pure preparation to a peptide sequencer, the first thirteen amino-terminal amino acids were determined to have the sequence Ala-Ser-Met-Thr-GlyGly-Gln-Gln-Met-Gly-Arg-Ser-Leu-. Thus the preparation was found to be a fused glicentin composed of native glicentin having attached to the amino terminus ten additional amino acids from the N-terminus of the s10 peptide.

### EXAMPLE 5

Preparation of native glicentin

The Met-Arg-fused glicentin was used as a substrate for cathepsin C.

15 mg of the substrate fused glicentin was dissolved in a solution containing 50 mM sodium acetate (pH 5.0), 5 mM NaCl and 5 mM DTT to a final concentration of 1 mg/ml. To this solution was added 3 units of cathepsin C using a 20 units/ml preparation manufactured by Boehringer Mannheim Yamanouchi. Furthermore, leupeptin was added to a final concentration of 25 µM to inhibit any nonspecific cleavage by contaminating proteases. After one hour of reaction at 22°C, the mixture was subjected to reversed phase HPLC on an ODS column to separate and recover the main peak with a retention time of 11.27 minutes. The separation conditions were as follows: rate of flow 1.0 ml/min; monitoring at 220 nm; Inertsil ODS-2 column (4.6 x 250 mm) manufactured by GL Science; elution in Solution A (20% acetonitrile + 0.002 M HCl) and Solution B (40% acetonitrile + 0.002 M HCl) with a linear gradient over a period of 20 minutes.

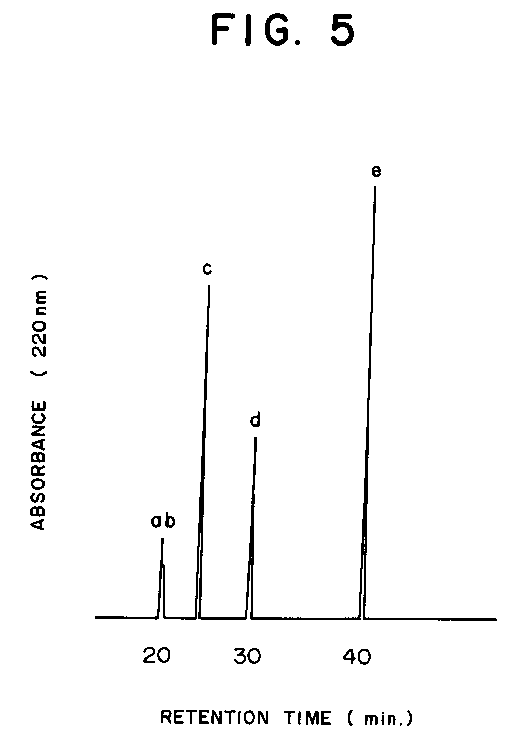

Analysis of the recovered major peak peptide with a peptide sequencer revealed that the amino-terminal sequence was Arg-Ser-Leu-Gln-Asp-Thr-, which is in agreement with that of native glicentin. In addition, the recovered major peak was cleaved in the conventional manner with lysyl endopeptidase manufactured by Boehringer Mannheim Yamanouchi, followed by HPLC separation for peptide mapping. The separation conditions were as follows: rate of flow 1.0 ml/min; monitoring at 220 nm; Inertsil ODS-2 column (4.6 x 250 mm) manufactured by GL Science; HPLC elution in Solution A (0.05% aqueous solution of trifluoroacetic acid) and Solution B (80% acetonitrile + 0.05% aqueous solution of trifluoroacetic acid) with a linear gradient over a period of 60 minutes from Solution A 100% / Solution B 0% to Solution A 20% / Solution B 80%. The HPLC chromatogram is shown in Fig. 5.

The respective peaks were separated and analyzed with a gas phase-type automatic peptide sequencer (PSQ-1, Shimazu Seisaku-Sho) to reveal that the peak fractions a, b, c, d and e had the amino acid sequences SEQ ID NOs: 14, 15, 16, 17 and 18, respectively, in the Sequence Listing, which corresponded to the regions 63-69, 1-9, 32-44, 10-31 and 45-62, respectively, of glicentin. The obtained peptide was confirmed to maintain the entire structure of native glicentin.

### EXAMPLE 6

Preparation of native glicentin

The s10-peptide-fused glicentin was used as a substrate for cathepsin C.

The substrate fused glicentin (15 mg) was dissolved in a solution containing 50 mM sodium acetate (pH 5.0), 5 mM NaCl and 5 mM DTT to a final concentration of 1 mg/ml. To this solution was added 3 units of cathepsin C using a 20 units/ml preparation manufactured by Boehringer Mannheim Yamanouchi. Furthermore, leupeptin was added to a final concentration of 25 µM to inhibit any nonspecific cleavage by contaminating proteases. After one hour of reaction at 22°C, the mixture was subjected to reversed phase HPLC to separate and recover the main peak. The separation conditions were the same as in Example 5.

Analysis with a peptide sequencer of the collected main peak peptide with a retention time of 11.44 minutes revealed that the amino-terminal sequence was Arg-Ser-Leu-Gln-Asp-Thr-, which is in agreement with that of native glicentin. In addition, the recovered main peak fraction was cleaved in the conventional manner with lysyl endopeptidase for peptide mapping. The HPLC chromatogram was the same as in Fig. 5. By analysis with a peptide sequencer of the amino acid sequence for each fragment, the peak was confirmed to retain the entire structure of native glicentin.

For comparison, methods of hydrolyzing with Factor Xa or enterokinase a fused glicentin composed of human glicentin having a peptide likewise attached at the amino-terminus are shown below as reference examples.

### REFERENCE EXAMPLE 1

There was hydrolyzed with Factor Xa a fused glicentin which was composed of human glicentin having attached at the amino-terminus a peptide of the sequence Ala-Ser-Met-Thr-Gly-Gly-Asn-Agn-Met-Gly-Asn-Gly-Ser-Ile-Glu-Gly-Arg. The substrate fused glicentin (100 µg) was dissolved in 200 µl of 2 mM CaCl₂-containing 20 mM Tris-HCl buffer (pH 8.0) and 0.08 unit of Factor Xa was added. After two hours of reaction at 28°C, HPLC was performed under the same conditions as in Example 5 to afford two reaction products. Analysis with a gas phase sequencer of the amino-terminal amino acid sequence of the products revealed that the sequence of the first ten amino-terminal amino acids of one product was Arg-Ser-Leu-Gln-Asp-Thr-Glu-Glu-Lys-Ser, which is in agreement with that of glicentin. The sequence of the first ten amino-terminal amino acids of the other product was Ser-Phe-Ser-Ala-Ser-Gln-Ala-Asp-Pro-Leu, which is in agreement with the sequence of the ten amino acids following the twelfth amino acid from the amino terminus of glicentin, Factor Xa causes hydrolysis of glicentin to take place between the eleventh amino acid Arg and the twelfth amino acid Ser, thereby giving rise to an incomplete glicentin with the first eleven amino-terminal amino acids deleted.

Changes with time in amounts of fused glicentin, glicentin and amino terminus-deleted glicentin formed during the reaction were checked. The results are shown in the following table.

**Table 1**

| Reaction time | Fused glicentin | Glicentin | Amino terminus-deleted glicentin |
|---|---|---|---|
| 1 hr | 56.8% | 32.1% | 11.1% |
| 2 hrs | 36.8 | 34.5 | 28.7 |
| 4 hrs | 2.5 | 33.5 | 64.0 |

The yield of glicentin formed by the reaction was less than 35% relative to the amount of the fused glicentin initially added, which shows that the glicentin production with Factor Xa is inferior to that with cathepsin C.

### REFERENCE EXAMPLE 2

The same fused glicentin as in Reference Example 1 was hydrolyzed with enterokinase. The substrate fused glicentin (100 µg) was dissolved in 1 ml of 10 mM CaCl₂- containing 40 mM sodium acetate buffer (pH 5.0), and 1 unit of enterokinase was added. After 3 hours of reaction at 37 °C, HPLC was performed under the same conditions as in Example 5 to detect cleavage into at least six fragments. Because of its nonspecific hydrolysis of glicentin, enterokinase is not suited for the production of glicentin.

SEQUENCE LISTING
SEQ ID NO: 1
SEQUENCE LENGTH: 19
SEQUENCE TYPE: nucleic acid
STRANDEDNESS: single
TOPOLOGY: linear
MOLECULE TYPE: synthetic DNA
SEQUENCE DESCRIPTION:
GATCCTTAGC GTAGGCCTT

SEQ ID NO: 2
SEQUENCE LENGTH: 19
SEQUENCE TYPE: nucleic acid
STRANDEDNESS: single
TOPOLOGY: linear
MOLECULE TYPE: synthetic DNA
SEQUENCE DESCRIPTION:
GATCAAGGCC TACGCTAAG

SEQ ID NO: 3
SEQUENCE LENGTH: 19
SEQUENCE TYPE: nucleic acid
STRANDEDNESS: double
TOPOLOGY: linear
MOLECULE TYPE: synthetic DNA
SEQUENCE DESCRIPTION:
GGATCCTTAG CGTAGGCCT

SEQ ID NO: 4

SEQUENCE LENGTH: 19

SEQUENCE TYPE: nucleic acid

STRANDEDNESS: single

TOPOLOGY: linear

MOLECULE TYPE: synthetic DNA

SEQUENCE DESCRIPTION:

CATGGCCCGG GACAGCACA

SEQ ID NO: 5

SEQUENCE LENGTH: 19

SEQUENCE TYPE: nucleic acid

STRANDEDNESS: single

TOPOLOGY: linear

MOLECULE TYPE: synthetic DNA

SEQUENCE DESCRIPTION:

AGCTTGTGCT GTCCCGGGC

SEQ ID NO: 6

SEQUENCE LENGTH: 23

SEQUENCE TYPE: nucleic acid

STRANDEDNESS: double

TOPOLOGY: linear

MOLECULE TYPE: synthetic DNA

SEQUENCE DESCRIPTION:

CCATGGCCCG GGACAGCAAG CTT

SEQ ID NO: 7
SEQUENCE LENGTH: 34
SEQUENCE TYPE: nucleic acid
STRANDEDNESS: single
TOPOLOGY: linear
MOLECULE TYPE: synthetic DNA
SEQUENCE DESCRIPTION:
AATTCAGATC TATCGAAGGT CGACGTTCTC TGCA

SEQ ID NO: 8
SEQUENCE LENGTH: 25
SEQUENCE TYPE: nucleic acid
STRANDEDNESS: single
TOPOLOGY: linear
MOLECULE TYPE: synthetic DNA
SEQUENCE DESCRIPTION:
GAGAACGTCG ACCTTCGATA GACTG

SEQ ID NO: 9
SEQUENCE LENGTH: 36
SEQUENCE TYPE: nucleic acid
STRANDEDNESS: double
TOPOLOGY: linear
MOLECULE TYPE: synthetic DNA
SEQUENCE DESCRIPTION:
10 20 30
GAATTCAGAT CTATCGAAGG TCGACGTTCT CTGCAG

SEQ ID NO: 10

SEQUENCE LENGTH: 18

SEQUENCE TYPE: nucleic acid

STRANDEDNESS: single

TOPOLOGY: linear

MOLECULE TYPE: synthetic DNA

SEQUENCE DESCRIPTION:

TATGCGTCGT TCCCTGCA

SEQ ID NO: 11

SEQUENCE LENGTH: 12

SEQUENCE TYPE: nucleic acid

STRANDEDNESS: single

TOPOLOGY: linear

MOLECULE TYPE: synthetic DNA

SEQUENCE DESCRIPTION:

GGGAACGACG CA

SEQ ID NO: 12

SEQUENCE LENGTH: 45

SEQUENCE TYPE: nucleic acid

STRANDEDNESS: single

TOPOLOGY: linear

MOLECULE TYPE: synthetic DNA

SEQUENCE DESCRIPTION:

TATGGCTAGC ATGACTGGTG GACAGCAAAT GGGTCGTTCC CTGCA

SEQ ID NO: 13
SEQUENCE LENGTH: 39
SEQUENCE TYPE: nucleic acid
STRANDEDNESS: single
TOPOLOGY: linear
MOLECULE TYPE: synthetic DNA
SEQUENCE DESCRIPTION:
GGGAACGACC CATTTGCTGT CCACCAGTCA TGCTAGCCA

SEQ ID NO: 14
SEQUENCE LENGTH: 7
SEQUENCE TYPE: amino acid
STRANDEDNESS: single
TOPOLOGY: linear
MOLECULE TYPE: peptide
SEQUENCE DESCRIPTION:
Arg-Asn-Arg-Asn-Asn-Ile-Ala

SEQ ID NO: 15
SEQUENCE LENGTH: 9
SEQUENCE TYPE: amino acid
STRANDEDNESS: single
TOPOLOGY: linear
MOLECULE TYPE: peptide
SEQUENCE DESCRIPTION:
Arg-Ser-Leu-Gln-Asp-Thr-Glu-Glu-Lys

SEQ ID NO: 16

SEQUENCE LENGTH: 13

SEQUENCE TYPE: amino acid

STRANDEDNESS: single

TOPOLOGY: linear

MOLECULE TYPE: peptide

SEQUENCE DESCRIPTION:

Arg-His-Ser-Gln-Gly-Thr-Phe-Thr-Ser-Asp-Tyr-Ser-Lys

SEQ ID NO: 17

SEQUENCE LENGTH: 22

SEQUENCE TYPE: amino acid

STRANDEDNESS: single

TOPOLOGY: linear

MOLECULE TYPE: peptide

SEQUENCE DESCRIPTION:

Ser-Arg-Ser-Phe-Ser-Ala-Ser-Gln-Ala-Asp-Pro-Leu-Ser-Asp-Pro-

Asp-Gln-Met-Asn-Glu-Asp-Lys

SEQ ID NO: 18

SEQUENCE LENGTH: 18

SEQUENCE TYPE: amino acid

STRANDEDNESS: single

TOPOLOGY: linear

MOLECULE TYPE: peptide

SEQUENCE DESCRIPTION:

Tyr-Leu-Asp-Ser-Arg-Arg-Ala-Gln-Asp-Phe-Val-Gln-Trp-Leu-Met-
Asn-Thr-Lys

## Claims

1. A process for the preparation of native human glicentin, which comprises producing by recombinant gene technology a fused polypeptide composed of human glicentin having an extra peptide moiety linked to the amino terminus and cleaving the extra peptide moiety in the fused polypeptide, wherein the peptide moiety upon cleavage is designed to constitute even-numbered amino acids and the cleavage of the extra peptide moiety is carried out by hydrolysis with cathepsin C.

2. A process of cliam 1 wherein the peptide moiety is T7S10 peptide.

3. A process of claim 1 wherein the peptide moiety consists of not greater than 20 amino acids.

4. A process of claim 1 wherein the amino acid number of the peptide moiety upon cleavage is rendered even by the construction of a gene encoding the following amino acid sequence Met-A-glicentin wherein Met stands for methionine encoded by the translation initiation codon ATG and A stands for a peptide consisting of an even number of amino acids, the amino terminus of which is selected from alanine, glycine, serine, valine or threonine to produce the fused polypeptide, by which the amino terminal methionine is removed through processing by a producing strain.

5. A process of claim 1 wherein the amino acid number of the peptide moiety upon cleavage is rendered even by the construction of a gene encoding the following amino acid sequence Met-B-glicentin wherein Met is as defined in claim 4 and B stands for either an amino acid selected from arginine, asparagine, aspartic acid, glutamine, glutamic acid, isoleucine, leucine, lysine, tryptophan, tyrosine, phenylalanine or hystidine, or a peptide consisting of an odd number of amino acid, the amino terminus of which is selected from arginine, asparagine, aspartic acid, glutamine, glutamic acid, isoleucine, leucine, lysine, tryptophan, tyrosine, phenylalanine or histidine to produce the fused polypeptide, by which the amino terminal methionine is not removed through processing by a producing strain.
